# EUROPEAN PATENT SPECIFICATION

(11) **EP 2 055 690 B1**
(45) Date of publication and mention of the grant of the patent: **19.07.2017**
(21) Application number: 07800769.7
(22) Date of filing: 23.08.2007
(51) Int. Cl.: C07C 1/24, C07C 1/20, C07C 11/04, C07C 11/06, C07C 43/04

(54) **A PROCESS FOR STARTING UP FLUIDIZED CATALYTIC REACTION APPARATUS USED FOR PRODUCING LOWER OLEFIN**
VERFAHREN FÜR DEN START EINER KATALYTISCHEN FLIESSBETTREAKTIONSVORRICHTUNG ZUR VERWENDUNG FÜR DIE HERSTELLUNG VON NIEDEREM OLEFIN
PROCÉDÉ DE DÉMARRAGE D'APPAREIL DE RÉACTION FLUIDISÉE POUR LA PRODUCTION D'OLÉFINES INFÉRIEURES

(30) Priority: 23.08.2006 CN 200610112558
(43) Date of publication of application: 06.05.2009
(73) Proprietor: Dalian Institute of Chemical Physics, Chinese Academy of Sciences, Dalian City, Liaoning Province 116023 (CN)
(72) Inventor: LIU, Zhongmin, Liaoning 116023 (CN); LV, Zhihui, Liaoning 116023 (CN); HE, Changqing, Liaoning 116023 (CN); LIU, Yu, Dalian, Liaoning 116023 (CN); QI, Yue, Liaoning 116023 (CN); MIN, Xiaojian, Dalian, Liaoning 116023 (CN); WANG, Gongwei, Liaoning 116023 (CN); WANG, Xiangao, Liaoning 116023 (CN); ZHANG, Jinling, Liaoning 116023 (CN)
(74) Representative: V.O.
(86) International application number: PCT/CN2007/002549
(87) International publication number: WO 2008/025254

(56) References cited:
- CN-A- 1 656 048
- US-B1- 6 187 983
- US-B1- 6 872 867

## Description

### FIELD OF THE INVENTION

This invention relates to a process for starting up a fluidized catalytic reaction apparatus used for producing lower olefins from methanol or/and dimethyl ether, which is suitable for the starting up of a circulating fluidized catalytic reaction apparatus of exothermic reaction type, and in particular, for the starting up of a fluidized catalytic reaction apparatus for producing lower olefins such as ethylene, propylene or the like from methanol or/and dimethyl ether.

### BACKGROUND

Ethylene and propylene are two basic raw materials with the largest consumption and many applications in chemical industry and are referred to as the stem of the modern organic synthesis industry, and therefore the production technology thereof is the emphasis to be developed competitively by the developed countries. The main route of producing the two olefins is light oil cracking and other methods include the catalytic conversion of lower alcohol ethers, aldehydes, mercaptans, and halohydrocarbons. The impacts of twice petroleum crises in 1970s accelerated the research and developing work of the technology for producing lower olefins through a non-petroleum raw material route wherein the process of methanol conversion has been developed rapidly and shows an enormous commercial application perspective.

Firstly, a process technology for producing lower olefins by taking ZSM-5 zeolite as catalyst and methanol as raw material was provided by Mobile Co., US.

Thereafter, in the middle of 1980s, an assumption of producing lower olefins with a catalyst of a non-zeolite type heteroatom containing aluminum phosphate salt molecular sieve was proposed by Union Carbide Corporation of USA (U.S. Pat. 4499327).

In 1990, a result of the conversion of methanol to lower olefins high selectively with a catalyst of a SAPO-34 type silicoaluminophosphate molecular sieve having a micropore structure of chabasite was published by Dalian Institute of Chemical Physics, Chinese Academy of Sciences for the first time (Applied Catalysis, 1990, Volume 64, P31-40). Subsequently, a new technology for synthesizing SAPO-34 molecular sieve, a preparation technology of SAPO-34 molecular sieve catalysts and a process technology for producing lower olefins from methanol or/and dimethyl ether were proposed (the patent numbers are CN1037334C, CN1038125C, CN1048429C, CN1065853C, CN1067603C and CN1076219C, respectively), and therefore the production costs of SAPO-34 molecular sieve and the catalyst thereof were reduced substantially, the yields of ethylene and propylene were increased prominently and the economic competitive power of the process technology approached to the level of the petroleum cracking technology.

As stepping into the 21^{st} century, in order to cope with the great pressure of scarce petroleum resources and the rapidly increasing oil price, the process technology for producing lower olefins from methanol or/and dimethyl ether using SAPO-34 molecular sieve catalyst developed by the applicant has been capable of satisfying the demand of industrial implementation.

Due to the small pore characteristic of SAPO-34 molecular sieve, this solid acid catalyst tends to be coked quickly and deactivated temporarily in a organic reaction and can be used only after a regeneration by carbon burning. In a continuous industrial production, the continuous stable operation of the small pore molecular sieve such as SAPO-34 or the like can be ensured only when a circulating fluidized apparatus including a reactor and a regenerator is used. And the design of an MTO process points to the use of a fluidized apparatus as the preferred commercial solution to the problem of efficiently and effectively using a SAPO type catalyst system in this type of device, such as U.S. Pat. No. 6,872,867 B1 which discloses a catalytic conversion process using a fluidized conversion zone, which requires a minimum superficial gas velocity to function properly, and a motor-driven, capacity-limited product compressor zone is started up using a thermal compressor by establishing two start-up gas recirculation circuits, one using the product compression zone running at high pressure to recirculate about 40 to 60 vol-% of the effluent gas stream from the conversion zone and the other running at low pressure and carrying the remaining portion of the effluent gas stream from the fluidized conversion zone where the high pressure circuit supplies motive gas to the thermal compression zone and the low pressure circuit supplies suction gas to the thermal compressor and the resulting compressed discharge gas enables the catalytic process to start up without the use of a dedicated motor-driven start-up compressor. In the fluidized apparatus, the temperature of the bed of the reactor for the conversion of methanol or/and dimethyl ether to lower olefins is 400 to 550°C and the temperature of the catalyst bed of the regenerator is 550 to 700°C. The circulating fluidized apparatus have a very common application in the process of petroleum fluid catalytic cracking (FCC). These apparatus have no heating components themselves and at the stage of starting up, the temperatures of the apparatus are increased depending by the external auxiliary heat-supplying equipments. In industry, such fluidized apparatus is very large in size and the filled catalyst at starting up is up to hundreds of tons, therefore very large amount of heat is needed to increase the bed temperatures of the reactor and the regenerator of the apparatus to 500°C or above, and especially when it is over 400°C or above, it is very difficult to increase the temperature by utilizing external heat.

A method commonly used in the process of FCC is that when the temperature of the regenerator catalyst bed attains 370°C or above, diesoline is spray into the bed and the temperature of the apparatus is elevated using the combustion exothermic reaction of the diesoline. The advantage of this method is that it can increase the temperature of the apparatus rapidly and reduce the starting up time greatly. At the same time, FCC is an endothermic reaction and the catalyst is needed to carry heat from the regenerator to maintain the temperature of the catalyst bed, therefore in the actual operation, fuel oil should be sprayed to the regenerator continuously to maintain the temperature of the regenerator.

However, this method has the following disadvantages: (1) a mass of diesel oil is consumed additionally; (2) at the initial stage of spraying oil, as the diesoline can not be burn completely, a mass of carbon black is produced and covered on the catalyst surface, and a part of the carbon black is flowed into the atmosphere with the tail gas and causes pollution to a certain extent; and (3) local superheating may occur so as to make the activity of part of catalyst lost permanently. As the process of the conversion of methanol or/and dimethyl ether to lower olefins is a strong exothermal reaction and the fluidized process of producing lower olefins from methanol or/and dimethyl ether has no precedents in industrial implement, especially SAPO-34 molecular sieve catalyst has not passed the test of actual industrial operation, whether the heating up method of spraying oil in FCC process is feasible or not is still unknown.

Therefore, how to utilize the characteristics of conversion reaction and start up the process is a challenge for this process in industrial implement.

### DISCLOSURE OF THE INVENTION

An object of the invention is to provide a process for starting up a fluidized catalytic reaction apparatus used for producing lower olefins from methanol or/and dimethyl ether.

In order to achieve the object described above, the invention provides a process for starting up a fluidized catalytic reaction apparatus for producing lower olefins, in which methanol or the mixture of methanol and dimethyl ether is taken as raw material, and when the catalyst bed of the circulating fluidized catalytic reaction apparatus is heated to 200°C by using a starting up auxiliary heat source, the raw material is fed to a reactor, whereby the heat released by the reaction of the raw material makes the temperature of the reactor increase quickly to a designed temperature, and after the coked catalyst in the reactor has been circulated to a regenerator, the coked catalyst is burned to release heat so as to increase the temperature of the regenerator to 540°C or above rapidly, consequently making the system reach the normal operation state rapidly.

The starting up process of the invention can also take dimethyl ether as raw material, and when the catalyst bed of circulating fluidized catalytic reaction apparatus is heated to 300°C by using a starting up auxiliary heat source, the raw material is fed to a reactor, whereby the heat released by the reaction of the raw material makes the temperature of the reactor increase quickly to a designed temperature, and after the coked catalyst in the reactor has been circulated to a regenerator, burning the coked catalyst to release heat so as to increase the temperature of the regenerator to 540°C or above rapidly, consequently making the system reach the normal operation state rapidly.

In an aspect of the invention, a process for starting up a fluidized catalytic reaction apparatus used for producing lower olefins is provided, wherein said catalytic reaction takes methanol, a mixture of methanol or dimethyl ether as raw material and said reaction apparatus includes a reactor having a catalyst bed and a regenerator having a regeneration bed, the process including the steps of:
1) when the catalyst bed is heated to 200°C or above when methanol or a mixture of methanol or dimethyl ether is taken as the raw material, or 300 °C or above when dimethyl ether is taken as the raw material by using a starting up auxiliary heat source, feeding the raw material to the reactor, whereby the heat released by the reaction of the raw material makes the temperature of the reactor increase quickly to 450 °C and coke the catalyst to produce a coked catalyst; and
2) circulating the coked catalyst of 450 °C in the reactor to the regeneration bed of the regenerator to accelerate the heating up of the generator, and when the temperature of the catalyst bed of the regenerator reaches 340 °C, the coked catalyst begins to burn, using burning of the coked catalyst to release heat so as to increase the temperature of the regenerator to 540°C or above rapidly, consequently making the system reach the normal operation state rapidly.

In the described process, the reaction apparatus is a circulating fluidized catalytic reaction apparatus consisting of a reactor and a regenerator.

In the described process, the catalyst in the catalyst bed of the reactor is a hydrogen type molecular sieve catalyst.

In the described process, the catalyst in the catalyst bed of the reactor is a solid acid catalyst.

Generally speaking, the invention provides a starting up process for an exothermic reaction type circulating fluidized process for the conversion of methanol or/and dimethyl ether to lower olefins, which can reduce the starting up cost, ensure the long-term stability of various solid acid catalysts, start up the production system rapidly and increase the economic benefits.

The embodiment of the invention is as follows: the circulating fluidized catalytic reaction apparatus is heated to 200°C or above by using a starting up auxiliary heat source, then a raw material of methanol or a mixture of methanol and dimethyl ether is fed to the reactor; the heat released by the reaction of the raw material makes the temperature of the reactor increase quickly to a designed temperature. The coked catalyst is burned in the regenerator so as to increase the temperature of the regenerator to 540°C or above rapidly, consequently making the system reach the normal operation state rapidly. When dimethyl ether is used as raw material for the reaction, the feeding can be performed only when the reaction apparatus has been heated to be 300°C or above.

In the embodiments described above, when the temperature of the reactor is between 200 to 300°C, a conversion reaction of methanol (CH₃OH) to dimethyl ether (CH₃OCH₃) takes place mainly in the catalyst bed and the heat released by the reaction increases the temperature of the reactor. The specific reaction is:

2CH₃OH → CH₃OCH₃ + H₂O -23.4 kJ/mol

The hydrogen type molecular sieve is a solid acid catalyst and under the effect thereof, the reaction mechanism is:

CH₃OH + H⁺B⁻ → CH₃OH₂⁺ + B⁻ → CH₃B + H₂O

CH₃B + CH₃OH → CH₃BCH₃OH → CH₃OCH₃ + H⁺B⁻

In this formulae, B⁻ represents the matrix of the molecular sieve.

After the temperature of the reactor reaches 300°C or above, the aforementioned conversion reaction of methanol to dimethyl ether and an exothermic conversion reaction of dimethyl ether to olefins ((CH₂)₂ₙ) are taken place:

nCH₃OCH₃ → (CH₂)₂ₙ + nH₂O -11 to 53 kJ/mol

The invention has the characteristics described below:
(1) The reaction apparatus is a circulating fluidized catalytic reaction apparatus consisting of a reactor and a regenerator;
(2) The catalyst is a hydrogen type molecular sieve catalyst or other solid acid catalysts;
(3) When the temperature of the catalyst bed of the reactor reaches 200°C or above, the raw material of methanol can be fed to initiate the reaction, and the heat produced by the reaction makes the temperature of the reactor increase quickly to a designed temperature and accelerate the heating up of the regenerator. When dimethyl ether is used as the raw material for the reaction, the feeding can be performed only when the reaction apparatus has been heated to be 300°C or above;
(4) The reaction of methanol conversion makes the catalyst to be coked, and after the coked catalyst has been entered into the regenerator with the circulation of the system and when the temperature of the catalyst bed of the regenerator reaches 340°C, the coked catalyst begins to burn and therefore the temperature of the regenerator is increased into a designed range rapidly; and
(5) By avoiding the use of the manner of heating up the regenerator by spraying diesoline to the catalyst bed of the regenerator and burning it, which must be employed at the starting up stage of traditional circulating fluidized catalytic reaction apparatus, this invention can shorten the starting up time and protect the catalyst, reduce the corresponding resource consumption and increase the economic benefits at the same time.

### DESCRIPTION OF THE DRAWINGS

Figure 1 is the process flow schematic diagram of the reaction-regeneration part in example 1.
Figure 2 is the temperature variation curves of the reaction bed and the regeneration bed at the heating up stage of an industrial amplifying apparatus with a methanol treating capacity of 60 tons /day and the variation curve of methanol conversion, according to the process provided in the invention.

### SPECIFIC MODE OF CARRYING OUT THIS INVENTION

After all the apparatuses used in the production system have been detected and confirmed to be in ready states, air is supplied into the regenerator and nitrogen gas is introduced into the reactor. The air and nitrogen gas are heated by using an external auxiliary heat source so as to realize the heating of the circulating fluidized apparatus. When the temperatures in the middle parts of the regenerator and the reactor are increased to 200°C or above, an active catalyst is added into the apparatus to a predetermined amount. Simultaneously, the amount of nitrogen gas and that of air are adjusted momentarily according to the temperatures of the reactor and the regenerator so as to make the catalyst being circulated between the reactor and the regenerator, and the cyclones of the reactor and the regenerator are insured to be able to work effectively so as to avoid the loss of a mass of catalyst.

When the temperature of the catalyst bed of the reactor is heated to 200°C and the temperature of the catalyst bed of the regenerator is heated to 300°C or above, the circulation of the catalyst is controlled to a state as low as possible and methanol is began to be fed into the reactor to initiate the methanol conversion reaction, and therefore the heat released by the reaction increases the temperature of the catalyst bed of the reactor rapidly. When the temperature of the catalyst bed of the reactor is increased to 450°C, the circulation amount of the catalyst is increased to stabilize the temperature of the reactor at 450°C, and simultaneously, a coked catalyst with a relatively high temperature is provided to the regenerator to accelerate the heating up of the catalyst bed of the regenerator. When the temperature of the catalyst bed of the regenerator reaches 340°C or above, the coked catalyst starts burning to accelerate the heating up of the catalyst bed of the regenerator continuously.

When the temperature of the catalyst bed of the generator increases to 540°C or above and the temperature of the catalyst bed of the reactor increases to a designed temperature, the operation parameters of the heat exchange, the addition of the reaction raw material, the circulation of catalyst and the like are adjusted to stabilize the temperatures of the reactor and the regenerator and the circulation amount of catalyst in designed adequate ranges so as to ensure the complete conversion of the raw material of the reaction and the relatively high selectivity for olefins.

When dimethyl ether is used as raw material, the implement process is substantially the same and the only difference is that the feeding can be performed only when the reaction apparatus has been heated to be 300°C or above.

The technical characteristics of the invention were introduced below by way of example, but the invention is not limited thereto.

### Example 1

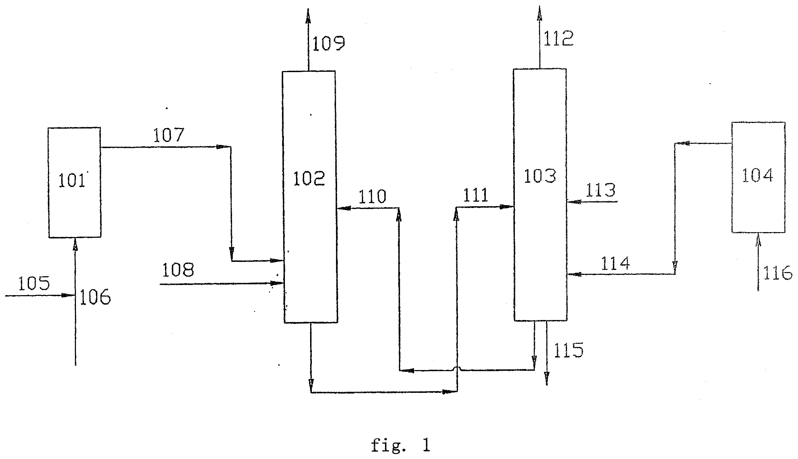

Fig. 1 is the process flow schematic diagram of the reaction-regeneration part in this example. 101 is a heater for pre-heating nitrogen gas or steam, 102 is a reactor, 103 is a regenerator, 104 is an auxiliary heater for pre-heating air, 105 is an inlet line for nitrogen gas, 106 is an inlet line for steam, 107 is a line for nitrogen gas or steam to enter the reactor, 108 is a feeding line for methanol, 109 is a line for product gas to a cooling system, 110 is a conveying line for the circulation of the catalyst after regeneration from the regenerator to the reactor, 111 is a conveying line for the circulation of the coked catalyst after reaction from the reactor to the regenerator, 112 is a discharging line for a regenerated fume, 113 is a conveying line for conveying catalyst from a catalyst storage tank to the regenerator, 114 is a line for conveying air from the auxiliary heater to the regenerator, 115 is a conveying line for returning catalyst from the regenerator to the catalyst storage tank, and 116 is a inlet line for air.

During the system operation, the total reserve of catalyst in the system was 1.2 to 1.6 times of the treating amount of methanol per hour. Reactor 102 was introduced with nitrogen gas through lines 105, 106, and 107, and the regenerator was introduced with air through lines 116 and 114. Subsequently, the heating apparatus 101 and 104 were started to heat nitrogen and air so that the reactor and regenerator were heated. When the middle part of the regenerator was increased to 502°C, the catalyst conveying apparatus was started and the addition of the catalyst into the regenerator through line 113 was started. During the catalyst addition, the flow rates of nitrogen gas and the air were adjusted momentarily according to the temperature variations of the reactor and the regenerator to make the cyclones of the reactor and the regenerator being capable of working effectively. Simultaneously, the opening degrees of the sliding valves at the bottoms of the reactor and the regenerator were adjusted so as to adjust the circulation amount of the catalyst.

After the addition of catalyst, the temperature of the catalyst bed of the reactor was reduced to be 149°C and the temperature of the catalyst bed of the regenerator was reduced to be 263°C, and the heating to the reactor and the regenerator were continued. When the temperature of the catalyst bed of the reactor was increased to be 271°C (the temperature of the bed of the regenerator is also increased to 319°C accordingly), the circulation amount of catalyst in the reactor and the regenerator were controlled to a state as low as possible firstly, and then methanol is fed into the bed of the reactor 102 through line 108 to start the methanol conversion reaction, thereby the reaction was started immediately. The feeding amount of methanol was increased gradually to ensure the complete conversion of methanol.

When the temperature of the catalyst bed of the reactor was 300°C or lower, the reaction product of methanol was mainly dimethyl ether and the catalyst has a very small coking amount. After the temperature of the bed has been increased to be 300°C or above, dimethyl ether began to be converted into hydrocarbons and the conversion was increased with the elevation of the temperature, and Simultaneously, the coking amount of catalyst was increased continuously. The conversion reactions of methanol to dimethyl ether and further to hydrocarbons were all strong exothermic and therefore the heating up speed of the reactor was accelerated.

When the temperature of the bed of the reactor was increased to 410°C, the temperature of the bed of the regenerator was also increased to be 360°C, the temperature of the catalyst bed of the regenerator was increased rapidly thereafter, that is, at this time, the cokes of the coked catalyst began to be burned in air flow automatically. According to the process described above, the temperatures of the catalyst beds of the reactor and the regenerator were increased to be 492 °C and 620 °C, respectively, and by further strengthening the measures of heat transfer and the like, the temperatures of the reactor and the regenerator were stabilized. Simultaneously, the stable operation of the system was realized by stabilizing the circulation amount of the catalyst and controlling the mass space rate of the fed methanol to be 5 h⁻¹.

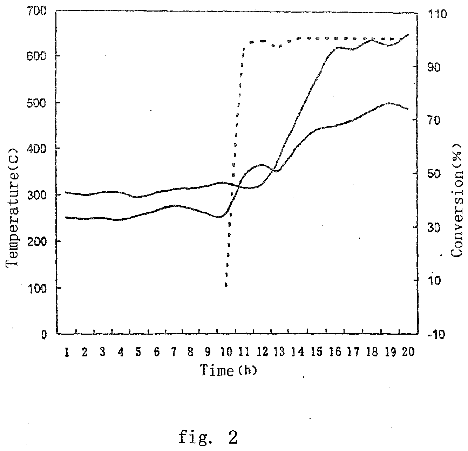

Fig. 2 is the temperature variation curves of the reaction bed and the regeneration bed at the heating up stage of an industrial amplifying apparatus with a methanol treating capacity of 60 tons /day and the variation curve of methanol conversion. In fig. 2, the broken line is the variation curve of methanol conversion and in the two solid lines, the solid line underlying on the left and right sides and superincumbent in the middle part is the temperature variation curve of the reaction bed and the solid line superincumbent on the left and right sides and underlying in the middle part is the temperature variation curve of the regeneration bed.

## Claims

1. A process for starting up a fluidized catalytic reaction apparatus used for producing lower olefins, wherein said catalytic reaction takes (1) methanol, (2) a mixture of methanol and dimethyl ether, or (3) dimethyl ether as raw material and said reaction apparatus includes a reactor having a catalyst bed and a regenerator having a regeneration bed, the method including the steps of:
1) when the catalyst bed is heated to 200 °C or above when (1) methanol or (2) a mixture of methanol and dimethyl ether is taken as the raw material, or 300 °C or above when (3) dimethyl ether is taken as the raw material by using a starting up auxiliary heat source, feeding the raw material to the reactor, whereby the heat released by the reaction of the raw material makes the temperature of the reactor increase quickly to 450 °C and coke the catalyst to produce a coked catalyst; and
2) circulating the coked catalyst of 450 °C in the reactor to the regenerator bed of the regenerator to accelerate the heating up of the regenerator, and when the temperature of the catalyst bed of the regenerator reaches 340 °C, the coked catalyst begins to burn, using burning of the coked catalyst to release heat so as to increase the temperature of the regenerator to 540 °C or above rapidly, consequently making the system reach the normal operation state rapidly.

2. The process as claimed in claim 1, **characterized in that** the reaction apparatus is a circulating fluidized catalytic reaction apparatus consisting of a reactor and a regenerator.

3. The process as claimed in claim 1, **characterized in that** the catalyst in the catalyst bed of the reactor is a hydrogen type molecular sieve catalyst.

4. The process as claimed in claim 1, **characterized in that** the catalyst in the catalyst bed of the reactor is a solid acid catalyst.

## Patentansprüche

1. Verfahren zur Inbetriebnahme einer fluidisierten, katalytischen Reaktionsvorrichtung, verwendet zur Herstellung niederer Olefine, wobei die katalytische Reaktion (1) Methanol, (2) ein Gemisch aus Methanol und Dimethylether oder (3) Dimethylether als Rohstoff nimmt und die Reaktionsvorrichtung einen Reaktor mit einem Katalysatorbett und einen Regenerator mit einem Regenerierungsbett beinhaltet, das Verfahren beinhaltend die Schritte von:
1) Wenn das Katalysatorbett auf 200 °C oder mehr erhitzt wird, wenn (1) Methanol oder (2) ein Gemisch aus Methanol und Dimethylether als das Rohmaterial genommen wird, oder 300 °C oder mehr, wenn (3) Dimethylether als das Rohmaterial genommen wird, unter Verwendung einer Anlaufhilfswärmequelle, Zuführen des Rohmaterials zu dem Reaktor, wobei die durch die Reaktion des Rohmaterials freigesetzte Hitze einen schnellen Anstieg der Temperatur des Reaktors auf 450 °C bewirkt und den Katalysator verkoksen lässt, um einen verkoksten Katalysator herzustellen; und
2) Zirkulieren des verkoksten Katalysators von 450 °C in dem Reaktor zu dem Regenerierungsbett des Regenerators, um das Erhitzen des Regenerators zu beschleunigen, und wenn die Temperatur des Katalysatorbetts des Regenerators 340 °C erreicht, der verkokste Katalysator zu verbrennen beginnt, Verwendung des Verbrennens des verkoksten Katalysators, um Hitze freizusetzen, sodass die Temperatur des Regenerators schnell auf 540 °C oder mehr erhöht wird, damit infolgedessen das System schnell den normalen Betriebszustand erreicht.

2. Verfahren wie beansprucht in Anspruch 1, **dadurch gekennzeichnet, dass** die Reaktionsvorrichtung eine zirkulierende, fluidisierte, katalytische Reaktionsvorrichtung ist, bestehend aus einem Reaktor und einem Regenerator.

3. Verfahren wie beansprucht in Anspruch 1, **dadurch gekennzeichnet, dass** der Katalysator in dem Katalysatorbett des Reaktors ein Molekularsiebkatalysator vom Wasserstofftyp ist.

4. Verfahren wie beansprucht in Anspruch 1, **dadurch gekennzeichnet, dass** der Katalysator in dem Katalysatorbett des Reaktors ein fester Säurekatalysator ist.

## Revendications

1. Procédé de démarrage d'un appareil de réaction catalytique fluidisée, utilisé pour la production d'oléfines inférieures, dans lequel ladite réaction catalytique fait intervenir (1) du méthanol, (2) un mélange de méthanol et de diméthyléther, ou (3) du diméthyléther en tant que matière première et ledit appareil de réaction comprend un réacteur ayant un lit catalytique et un régénérateur ayant un lit de régénération, le procédé comprenant les étapes consistant à :
1) quand le lit catalytique est chauffé à 200 °C ou plus quand (1) le méthanol ou (2) un mélange de méthanol et de diméthyléther est pris comme matière première ou à 300 °C ou plus quand (3) le diméthyléther est pris comme matière première, en utilisant une source de chaleur auxiliaire de démarrage, charger la matière première dans le réacteur, moyennant quoi la chaleur libérée par la réaction de la matière première fait augmenter rapidement la température du réacteur jusqu'à 450 °C et cokéfie le catalyseur pour produire un catalyseur cokéfié ; et
2) faire circuler le catalyseur cokéfié à 450 °C dans le réacteur jusqu'au lit régénérant du régénérateur pour accélérer le chauffage du régénérateur et quand la température du lit catalytique du régénérateur atteint 340 °C, le catalyseur cokéfié commence à brûler, utiliser la calcination du catalyseur cokéfié pour libérer de la chaleur de manière à porter rapidement la température du régénérateur jusqu'à 540 °C ou plus, ce qui permet par conséquent au système de parvenir à l'état de fonctionnement normal rapidement.

2. Procédé selon la revendication 1, **caractérisé en ce que** l'appareil de réaction est un appareil de réaction catalytique fluidisée circulant se composant d'un réacteur et d'un régénérateur.

3. Procédé selon la revendication 1, **caractérisé en ce que** le catalyseur dans le lit catalytique du réacteur est un catalyseur à tamis moléculaire de type hydrogène.

4. Procédé selon la revendication 1, **caractérisé en ce que** le catalyseur dans le lit catalytique du réacteur est un catalyseur acide solide.
